(19) Europäisches Patentamt / European Patent Office / Office européen des brevets

(11) **EP 2 447 867 B1**

(12) **EUROPEAN PATENT SPECIFICATION**

(45) Date of publication and mention of the grant of the patent:
**11.12.2024 Bulletin 2024/50**

(51) International Patent Classification (IPC):
**G16C 60/00** *(2019.01)*     G16C 20/30 *(2019.01)*
**G16C 10/00** *(2019.01)*

(21) Application number: **10189399.8**

(52) Cooperative Patent Classification (CPC):
**G16C 60/00;** G16C 10/00; G16C 20/30

(22) Date of filing: **29.10.2010**

(54) **Method for determining the performances of a superabsorbent polymer material**

Verfahren zur Bestimmung der Leistungen eines hochabsorbierenden Polymermaterials

Procédé pour déterminer les performances d'un matériau polymère superabsorbant

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.05.2012 Bulletin 2012/18**

(73) Proprietor: **The Procter & Gamble Company Cincinnati, OH 45202 (US)**

(72) Inventors:
  • **Verstraete, Pierre**
    **1853 Strombeek-Bever (BE)**
  • **Lindner, Torsten**
    **61476 Kronberg (DE)**
  • **Meyer, Axel**
    **60486 Frankfurt am Main (DE)**
  • **Mattias, Schmidt**
    **65510 Idstein (DE)**
  • **Grass, Kai**
    **60486 Frankfurt am Main (DE)**
  • **Holm, Christan**
    **70569 Stuttgart (DE)**

(74) Representative: **P&G Patent Germany Procter & Gamble Service GmbH Sulzbacher Straße 40 65824 Schwalbach am Taunus (DE)**

(56) References cited:
**WO-A2-03/079946**

• **ARNOLD A. ET AL: "Simulating Charged Systems with ESPResSO",** COMPUTER SIMULATIONS IN CONDENSED MATTER SYSTEMS : FROM MATERIALS TO CHEMICAL BIOLOGY; [LECTURE NOTES IN PHYSICS], BERLIN [U.A.] : **SPRINGER, DE, vol. 703, 1 January 2006 (2006-01-01), pages 193 - 222, XP008133226, ISBN: 978-3-540-35270-9**

• **MANN B. A. ET AL: "The swelling behavior of charged hydrogels",** MACROMOL. SYMP., **SPECIAL ISSUE: MOLECULAR MOBILITY AND ORDER IN POLYMER SYSTEMS, vol. 237, no. 1, 2006, pages 90 - 107, XP002623544, ISBN: 3-527-31743-0**

• **PADDING J. T. ET AL: "Time and length scales of polymer melts studied by coarse-grained molecular dynamics simulations",** JOURNAL OF CHEMICAL PHYSICS, AMERICAN INSTITUTE OF PHYSICS, NEW YORK, NY, US, **vol. 117, no. 2, 8 July 2002 (2002-07-08), pages 925 - 943, XP008133117, ISSN: 0021-9606, DOI: 10.1063/1.1481859**

• **LIMBACH H. J. ET AL: "ESPResSo-an extensible simulation package for research on soft matter systems",** COMPUTER PHYSICS COMMUNICATION, ELSEVIER SCIENCE PUBLISHERS B.V., AMSTERDAM, NL, **vol. 174, no. 9, 1 May 2006 (2006-05-01), pages 704 - 727, XP025016803, ISSN: 0010-4655, [retrieved on 20060501], DOI: 10.1016/J.CPC.2005.10.005**

• **ANDERSON J. A. ET AL: "General purpose molecular dynamics simulations fully implemented on graphics processing units",** JOURNAL OF COMPUTATIONAL PHYSICS, LONDON, GB, **vol. 227, no. 10, 8 February 2008 (2008-02-08), pages 5342 - 5359, XP022577514, ISSN: 0021-9991**

- **MANN B. A. ET AL: "Swelling of polyelectrolyte networks", THE JOURNAL OF CHEMICAL PHYSICS, vol. 122, no. 15, 1 January 2005 (2005-01-01), pages 154903/1 - 14, XP009501494, ISSN: 0021-9606, DOI: 10.1063/1.1882275**

**Description**

FIELD OF THE INVENTION

The present invention is directed to a method for determining the performances of a superabsorbent polymer material by using a virtual model of the superabsorbent polymer material.

BACKGROUND OF THE INVENTION

Absorbent articles, such as disposable diapers, training pants, and adult incontinence undergarments, absorb and contain body exudates. Some absorbent articles, like diapers, contain absorbent polymer materials also known as superabsorbent polymer materials.

Superabsorbent polymer materials are able to absorb liquid and swell when entering into contact with liquid exudates. However, it has been shown in the past that not all categories of superabsorbent polymer materials are suitable for use in an absorbent article.

Indeed, superabsorbent polymer materials not only need to absorb large amount of liquids, but they also need to maintain their shape during the swelling process. During the swelling process, superabsorbent polymer materials typically form a gel. However, it may happen that the gel forms a seal in the top layer of the absorbent article, and thus prevent further liquid to reach the unutilized superabsorbent polymer materials in the lower layers. This phenomenon called "gel blocking" may induce leakages in the absorbent article. Therefore, it is important to have superabsorbent polymer materials which are strong enough and to avoid that the gel becomes "too soft", i.e. that the gel conforms too much after liquid uptake.

Hence, superabsorbent polymer materials particularly suitable for use in absorbent articles should consequently exhibit high absorption capacity while simultaneously maintain high gel strength.

A so-called capacity/gel strength trade off curve representing the variation of the gel strength with the absorption capacity is typically generated in order to select the most suitable superabsorbent polymer material with the better trade-off between capacity and gel strength. Any improvement of this trade-off curve enables better performance or cost savings of absorbent articles comprising such superabsorbent polymer materials.

The absorption capacity and the gel strength of the superabsorbent polymer materials may depend on different molecular parameter(s) of the materials. For example, the absorption capacity and gel strength of the materials may depend on the fraction of cross-linker present during the polymerization process. Indeed, the higher the fraction of cross-linker the lower the capacity and the higher the gel strength.

Up to now, testing the influence of different molecular parameters of the superabsorbent polymer materials on the performance parameter(s) of the superabsorbent polymer materials such as the absorption capacity and gel strength has required the manufacturing of different samples of superabsorbent polymer materials, each of them having different values for the molecular parameter of interest. The manufacturing of such superabsorbent polymer materials can be quite challenging since it is important to make sure that during the synthesis, only the tested parameter is varying. Otherwise, interpreting the results on capacity and gel strength can be more difficult. In addition, the manufacturing of superabsorbent polymer materials can considerably increase the development costs.

Thus, there is a need for a reliable and cost effective method which enables the testing of the influence that different molecular parameters of superabsorbent polymer materials may have on the performance parameter(s) of such materials.

Thereto, the inventors have found that molecular dynamics simulations can be used for this purpose.

The inventors have developed a method using a coarse-grained molecular dynamics model in order to test the influence of different molecular parameters of superabsorbent polymers on the performance of such polymers in a reliable, time- and cost-effective manner since no synthesis of these materials is consequently needed for testing. Once the appropriate value(s) of the molecular parameter(s) have been determined by the method, only the superabsorbent polymer materials having such value(s) for the molecular parameter(s) can be synthesized.

Articles related to the present invention are: Arnold A. et al. (2006) "Simulating Charged Systems with ESPRESSO", Computer Simulations in Condensed Matter Systems: from Materials to Chemical Biology; Lecture Notes in Physics, vol. 703, pages 193-222; and Mann B. A. et al. (2006) "The swelling behavior of charged hydrogels", Macromol. Symp., Special issue: Molecular Mobility and Order in Polymer Systems, vol. 237, no. 1, pages 90-107; and Padding J. T. et al. (2002) "Time and length scales of polymer melts studies by coarse-grained molecular dynamics simulations", J. Chem. Phys., vol. 117, no. 2, pages 925-943; as well as Mann B. A. et al. (2005) "Swelling of polyelectrolyte networks", The Journal of Chemical Physics, vol. 122, no. 15, pages 154903/114.

SUMMARY OF THE INVENTION

A method for determining the performances of a superabsorbent polymer comprises the steps of:

a) inputting the value(s) of one or more first molecular parameter(s) of the superabsorbent polymer into a coarse-grained molecular dynamics model; and calculating the value(s) of one or more first performance output parameter(s); and

b) inputting the value(s) of one or more second molecular parameter(s) of the superabsorbent polymer into said coarse-grained molecular dynamics model; and calculating the value(s) of one or more second performance output parameter(s); and

c) determining the variation between the value(s) of the one or more first performance output parameter(s) and the value(s) of the one or more second performance output parameter(s),

wherein a computer system is used for operating the coarse-grained molecular dynamics model; and

wherein the method is used for obtaining superabsorbent polymer materials suitable for use in absorbent articles; and further

wherein the value(s) of one or more first molecular parameter(s) inputted in step a), form a first input value set of a first molecular parameter set, and the value(s) of one or more second molecular parameter(s) inputted in step b), form a second input value set of a second molecular parameter set; and

wherein said first and second molecular parameter sets are the same and comprise molecular parameters selected from the group consisting of: cross-linker density, polydispersity index, percentage of dangling chains, degree of neutralization, functionality of the cross-linker molecules, percentage of extractable, molecular weight of the monomers and combinations thereof, and

wherein the value(s) of one or more first performance output parameters calculated in step a), form a first output value set of a first output parameter set, and the value(s) of one or more second performance output parameters calculated in step b), form a second output value set of a second output parameter set, and wherein said first and second output parameter sets comprise performance output parameters selected from the group consisting of: i) swelling capacity, ii) bulk modulus, iii) shear modulus and combinations thereof.

The variation determined in step c) is determined between different values of the same first and second performance output parameter.

In some embodiments, the computer system used in the method of the present invention may have a central processing unit, a graphical user interface including a display communicatively coupled to the central processing unit, and a user interface selection device communicatively coupled to the central processing unit.

BRIEF DESCRIPTION OF THE DRAWINGS

Fig. 1 is a representation of a portion of a polyacrylate polymer under the coarse-grained molecular dynamics approach.

Fig. 2 is a representation of a cubic simulation cell comprising a superabsorbent polymer as displayed by the LAMMPS software.

DETAILED DESCRIPTION OF THE INVENTION

"Superabsorbent polymer material" is used herein to refer to a superabsorbent polymer material which may be obtained by any polymerization process known by the person skilled in the art.

"Superabsorbent polymer" is used herein to refer to a superabsorbent polymer material which is simulated according to the coarse-graining approach.

A superabsorbent polymer and a superabsorbent polymer material can typically absorb at least 10 times its weight of an aqueous 0.9% saline solution as measured using the Centrifuge Retention Capacity test (EDANA WSP 241.2 (05)).

A superabsorbent polymer material may be in particulate form so as to be flowable in the dry state. This may be any polymeric material with such a Centrifuge Retention Capacity, such as starch-based superabsorbent polymer materials, or in some embodiment this material may be or include poly(meth)acrylic acid/ poly(meth)acrylate polymer materials, or preferably polyacrylic acid/polyacrylate polymer materials.

"Absorbent articles" is used herein to refer to devices that absorb and contain body exudates, and, more specifically, refers to devices that are placed against or in proximity to the body of the wearer to absorb and contain the various exudates discharged from the body. Absorbent articles include diapers, training pants, adult incontinence undergarments, feminine hygiene products and the like.

The coarse-grained molecular dynamics model

The present method uses a coarse-grained molecular dynamics model to simulate a superabsorbent polymer material.

The superabsorbent polymer of the model comprises polyelectrolyte polymer chains of polymerized monomers connected to uncharged cross-linker molecules. In such a model, the atomistic details of the polyelectrolyte polymer chains and of the cross-linker molecules are coarse-grained out and represented by spherical beads.

The chains are charged depending on the degree of neutralization of the superabsorbent polymer network. The charged monomers of the polyelectrolyte polymer chains are represented by charged spherical beads with the electric charges of the beads being placed at the center of the beads. The spherical beads representing the monomers of a polyelectrolyte polymer chain are connected by non-linear elastic springs.

In some embodiments, the superabsorbent polymer further comprises counterions. In such embodiments, the counterions are also represented by charged spherical beads with the electric charges of the beads being also placed at the center of the beads.

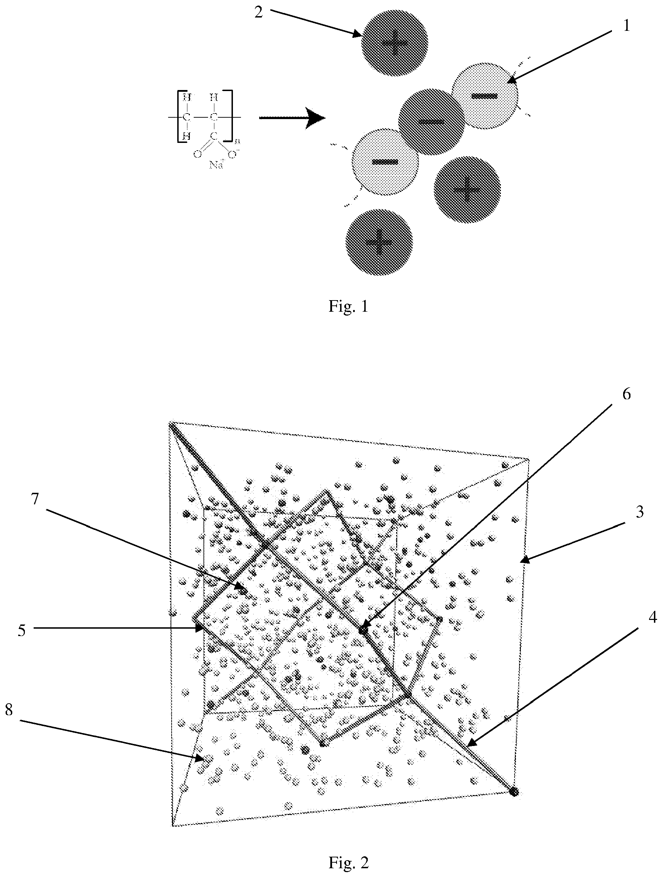

In some embodiments, the superabsorbent polymer is a cross-linked sodium polyacrylate polymer. In such embodiments, as for example shown on Figure 1, the monomers are polymerized acrylate monomers 1 which are represented by negatively-charged spherical beads with the negative charge being placed at the center of the beads and the counterions are sodium ions 2 which are represented by positively-charged spherical beads with the positive charge being placed at the center of the beads.

In the embodiments wherein the performances of the superabsorbent polymer are determined for a swollen gel of the superabsorbent polymer which is contained in a saline solution, the sodium and the chloride ions are also represented by charged spherical beads with the electric charges of the beads being also placed at the center of the beads.

In some embodiments, the superabsorbent polymer is represented by a diamond lattice comprising 16 polyelectrolyte polymer chains which are connected to 8 tetra-functional cross-linker molecules. In some of these embodiments, the superabsorbent polymer is further neutralized by adding the required amount of counterions such as the sodium ions.

In some other embodiments, the superabsorbent polymer is represented by a regular cubic lattice comprising 24 polyelectrolyte polymer chains which are connected to 8 hexa-functional cross-linker molecules. In some of these embodiments, the superabsorbent polymer is further neutralized by adding the required amount of counterions such as the sodium ions.

In some other embodiments, the superabsorbent polymer is represented by a regular cubic centered lattice comprising 64 polyelectrolyte polymer chains which are connected to 16 octafunctional cross-linker molecules. In some of these embodiments, the superabsorbent polymer is further neutralized by adding the required amount of counterions such as the sodium ions.

The coarse-grained molecular dynamics simulation uses reduced simulation units (r.s.u.) for all physical observables, i.e. they are dimensionless when expressed in terms of some scale parameters. The scales for length and energy quantities are respectively $\sigma$ and $\varepsilon$, which depend on the chosen model parameters.

The equilibrium bond-length between the polymerized monomers of the polyelectrolyte polymer chains of the model is determined by the parameters describing the below mentioned potentials (i.e. the truncated Lennard-Jones, the FENE and the coulomb potential). The equilibrium bond-length is $b_0 = 0.9$ r.s.u.

• Interactions:

According to the model of the present invention, the polymerized monomers of the polyelectrolyte polymer chains interact with one another intra and inter molecular, with the cross-linker molecules and with the counterions (when present) via a truncated (and shifted) Lennard-Jones (or Weeks-Chandler-Anderson) potential as defined in the following formula:

$$U_{\mathrm{LJ}}(r_{ij} < r_{\mathrm{cut}}) = 4\epsilon \left[ \left( \frac{\sigma}{r_{ij}} \right)^{12} - \left( \frac{\sigma}{r_{ij}} \right)^{6} + \frac{1}{4} \right]$$

F1

wherein $\sigma$ is the length parameter, $\varepsilon$ the energy parameter, $r_{ij}$ the distance between the centres of two beads i and j and

$r_{cut}$ the cut-off distance beyond which the interaction potential is zero.

In the embodiments wherein the performances of the superabsorbent polymer are determined for a swollen gel of the superabsorbent polymer which is contained in a saline solution, the polymerized monomers of the polyelectrolyte polymer chains also interact with the ions of the saline solution via the above-mentioned truncated (and shifted) Lennard-Jones (or Weeks-Chandler-Anderson) potential.

In some embodiments, the parameters are chosen to be: $\varepsilon$ = 1.0 r.s.u, $\sigma$ = 1.0 r.s.u. and $r_{cut}$ = $2^{1/6}$ = 1.12246 r.s.u.

The polymerized monomers of a same polyelectrolyte polymer chain interact with one another and with the cross-linker molecules to which the chain is connected via a FENE (Finitely Extendible Nonlinear Elastic) potential as defined in the following formula:

$$U(r_{ij}) = -\frac{1}{2}KR_{\mathrm{max}}^2 \ln\left[1 - \left(\frac{r_{ij} - r_0}{R_{\mathrm{max}}}\right)^2\right]_{F2}$$

wherein $r_{ij}$ the distance between the centre of two beads i and j, K is the force constant, $r_0$ the bond length at equilibrium and $R_{max}$ the bond length maximum.

In some embodiments, the parameters are chosen to be: K = 10.0 r.s.u., $R_{max}$ = 1.5 r.s.u. and $r_0$ = 0.0.

The polymerized monomers of the polyelectrolyte polymer chains of the model interact with one another intra and inter molecular and with the counterions (when present) via a coulomb potential as defined in the following formula F3:

$$U_{\mathrm{C}}(r_{ij}) = l_B k_B T \frac{q^2}{r_{ij}}_{F3}$$

Wherein q is the charge which is carried by all charged elements of the system (chain monomers / counterions (when present)), $k_B T$ is the temperature of the system, $l_B$ is the Bjerrum length, $r_{ij}$ the distance between the centre of two beads i and j.

The Bjerrum length defines the length scale at which the strength of the electrostatic interactions between two charges in a system is exactly equal to the thermal energy in the system: $l_B = e^2/(4\pi\varepsilon_0\varepsilon_r k_B T)$, where e is the elementary charge, $\varepsilon_0$ is the dielectric permittivity of the vacuum and $\varepsilon_r$ the relative permittivity.

The counterions also interact with one another via a coulomb potential as defined in the above mentioned formula F3.

In the embodiments wherein the superabsorbent polymer is contained in a saline solution, the chloride and sodium ions of the saline solution also interact with one another, with the polymerized monomers of the polyelectrolyte polymer chains and with the counterions via a coulomb potential as defined in the above mentioned formula F3.

In some embodiments, the parameters are chosen to be: q = $\pm$1 r.s.u., $k_B T$= 1.0 r.s.u. and $l_B$=2.58 r.s.u.

The coulomb interaction is calculated using the P3M (Particle-Particle-Particle-Mesh) algorithm under 3D periodic boundary conditions with metallic surface term and an accuracy of $10^{-2}$.

• Conversion between reduced and real-world units:

As mentioned above, the length-scale $\sigma$ and the energy-scale $\varepsilon$ of the model are expressed in reduced simulation units (r.s.u.). However, these two parameters as well as additional parameters such as the volume and the pressure of the model can also be converted into real-world units.

In the model, the equilibrium bond-length between the polymerized monomers of the polyelectrolyte polymer chains is $b_0$ = 0.9 r.s.u. In the real-world, the distance between two polymerized acrylate monomers of a polyacrylate polymer material is approximately 2.5 Angstrom. The ratio between these two values gives the length-scale expressed in real-world units.

$$\sigma = \frac{2.5\text{Å}}{0.9} = 2.78\text{Å}$$

In the model, the system is simulated at constant room temperature (~ 300K) using a Langevin thermostat with friction $\Gamma$ = 1.0 and temperature T= 1.0. The energy-scales are expressed in real-world units as follow:

$$\epsilon = k_{\mathrm{B}}T = 1.38 \times 10^{-23}\,\mathrm{J/K} \cdot 300\mathrm{K} = 4.14 \times 10^{-21}\,\mathrm{J}$$

Volume: The conversion between reduced simulation units and real-world units for the volume is:

$$1\mathrm{r.s.u.} \equiv \sigma^3 = 21.4 \times 10^{-30}\,\mathrm{m}^3$$

Pressure: The conversion between reduced simulation units and real-world units for the pressure (and analogously for the bulk modulus) is:

$$1\mathrm{r.s.u.} \equiv \frac{\epsilon}{\sigma^3} = 1.93 \times 10^8\,\mathrm{Pa}$$

Description of the method

The method according to the present invention enables the determination of the performances of a superabsorbent polymer.

In this method, the value(s) of one or more first molecular parameter(s) of a superabsorbent polymer are inputted into a coarse-grained molecular dynamics model and the value(s) of one or more first performance output parameter(s) are then calculated. The value(s) of one or more second molecular parameter(s) of a superabsorbent polymer are inputted into the coarse-grained molecular dynamics model and the value(s) of one or more second performance output parameter(s) are then calculated.

The variation between the value(s) of the one or more first performance output parameter(s) and the value(s) of the one or more second performance output parameter(s) is then determined.

As soon as a superabsorbent polymer or a superabsorbent polymer material enters into contact with a liquid it starts to swell. During the swelling process, the superabsorbent polymer or the superabsorbent polymer material typically forms a gel. The superabsorbent polymer or the superabsorbent polymer material swells until the swelling equilibrium is reached. At the swelling equilibrium, the amount of liquid absorbs by the superabsorbent polymer or the superabsorbent polymer material corresponds to the swelling capacity of the superabsorbent polymer or of the superabsorbent polymer material.

"Swelling gel" is used herein to refer to the gel of the superabsorbent polymer until the swelling equilibrium is reached.

"Swollen gel" is used herein to refer to the gel of the superabsorbent polymer at the swelling equilibrium.

In some embodiments, the performances of the superabsorbent polymer are determined for a swollen gel of the superabsorbent polymer which is contained in deionized water.

In some other embodiments, the performances of the superabsorbent polymer are determined for a swollen gel of the superabsorbent polymer which is contained in a saline solution. In some embodiments, the saline solution is a 0.01 to 5 w% saline solution (sodium chloride solution). In some other embodiments, the saline solution is a 0.9 w% saline solution.

The value(s) of one or more first molecular parameter(s) inputted into the coarse-grained molecular dynamics model, form a first input value set of a first molecular parameter set, and the value(s) of one or more second molecular parameter(s) inputted into the coarse-grained molecular dynamics model, form a second input value set of a second molecular parameter set. The first and second molecular parameter sets are the same. The first and the second molecular parameter sets comprise molecular parameters which are selected from the group consisting of: cross-linker density, polydispersity index, percentage of dangling chains, degree of neutralization, functionality of the cross-linker molecules, percentage of extractable, molecular weight of the monomers and combinations thereof.

It should be understood for the purpose of the invention that in order to be able to determine the influence that one of the molecular parameters may have on the performance output parameters, only the value of one same molecular parameter has to differ between the first input value set and the second input value set.

In some embodiments, the first input value set and the second input value set comprise the values of the cross-linker density.

In some embodiments, additional input value sets may be inputted into the coarse-grained molecular dynamics model for which the value(s) of one or more performance output parameter(s) is/are calculated.

The molecular parameter(s) (input parameters)

• The cross-linker density

The cross-linker density is defined as the ratio of the number of cross-linker molecules over the number of polymerized monomers of a superabsorbent polymer or of a superabsorbent polymer material.
In the embodiments wherein both the first and the second molecular parameter sets comprise the cross-linker density, the values of the cross-linker density may range between 0.01 to 2 mol%.

• The Polydispersity Index

The term "polydispersity" is used herein to refer to the polydispersity of polyelectrolyte polymer chains which are disposed between two cross-linker molecules in a superabsorbent polymer or a superabsorbent polymer material.
The polydispersity index (PDI) is a measure of the distribution of molecular weights in a superabsorbent polymer or in a superabsorbent polymer material. The PDI is calculated by dividing the weight average molecular weight ($M_w$) of the superabsorbent polymer or of the superabsorbent polymer material by the number average molecular weight ($M_n$) of respectively the superabsorbent polymer or the superabsorbent polymer material. The PDI is denoted as:

$$PDI = \frac{M_w}{M_n} \text{ wherein } M_n = \frac{\sum M_i N_i}{\sum N_i} \text{ and } M_w = \frac{\sum M_i^2 N_i}{\sum M_i N_i}$$

wherein $M_i$ is the molecular weight of a polyelectrolyte polymer chains of the superabsorbent polymer or of the superabsorbent polymer material comprising i monomers and $N_i$ is the number of polyelectrolyte polymer chains comprising i monomers.
In the embodiments wherein both the first and the second molecular parameter sets comprise the polydispersity index, the values of the polydispersity index may range between 1 and 5.

• The percentage of dangling chains

Dangling chains are imperfections of the superabsorbent polymer or of the superabsorbent polymer material network. In some embodiments, dangling chains are created by cutting the polymer chains of the superabsorbent polymer material at the cross-linker molecules. In some other embodiments, dangling ends are created by cutting the chains in the middle, thereby creating two dangling ends per cut.
In the embodiments wherein both the first and the second molecular parameter sets comprise the percentage of dangling chains, the percentage of dangling chains may range between 0% and 50%, preferably between 0% and 25%.

• Degree of neutralization

In some embodiments, the superabsorbent polymer or the superabsorbent polymer material network is neutralized. In some of these embodiments, the superabsorbent polymer or the superabsorbent polymer material network is neutralized with a sodium hydroxide solution. The degree of neutralization of a superabsorbent polymer or a superabsorbent polymer material corresponds to the percentage of polymerized monomers of respectively the superabsorbent polymer or the superabsorbent polymer material being negatively charged. For example, if the degree of neutralization is of 75 mol%, 75 mol% of the monomers are negatively charged and 25 mol% of the monomers are neutral.
In the embodiments wherein both the first and the second molecular parameter sets comprise the degree of neutralization, the values of the degree of neutralization may range between 0 and 100 mol%. In some preferred embodiments, the values of the degree of neutralization may range between 50 and 100 mol%.

• Functionality of the cross-linker molecules

In the embodiments wherein both the first and the second molecular parameter sets comprise the functionality of the cross-linker molecules, the cross-linker molecules may be tetrafunctional, hexafunctional or octafunctional.
In some embodiments, the superabsorbent polymer material may comprise tetrafunctional cross-linker molecules such as polyethyleneglycol di(meth)acrylate or methylenebisacrylamide, hexafunctional cross-linker molecules such as triallylamine and/or octafunctional cross-linker molecules such as tetraallyloxyethane.

• The percentage of extractable

The percentage of extractable of a superabsorbent polymer or of a superabsorbent polymer material is defined as the weight percentage of respectively the superabsorbent polymer or the superabsorbent polymer material which is soluble in the liquid in which the superabsorbent polymer or the superabsorbent polymer material is contained. In other words, the percentage of extractable corresponds to the weight percentage of superabsorbent polymer or superabsorbent polymer material which is not retained in respectively the superabsorbent polymer or the superabsorbent polymer material network. In the embodiments wherein both the first and the second molecular parameter sets comprise the percentage of extractable, the percentage of extractable may range between 0 and 50%, preferably between 0 and 15%:

• Molecular weight of the monomers

In the embodiments wherein both the first and the second molecular parameter sets comprise the molecular weight of the monomers, the values of the molecular weight may range between 28 to 72 g/mol.

The values of one or more first performance output parameters are calculated forming a first output value set of a first output parameter set. The values of one or more second performance output parameters are also calculated forming a second output value set of a second output parameter set. The first and the second output parameter sets are selected from one of the following parameters: Swelling Capacity, bulk modulus, shear modulus and combinations thereof.

The performance output parameters:

• swelling capacity

The swelling capacity of a superabsorbent polymer or a superabsorbent polymer material refers to the maximum amount of swelling solution in which respectively the superabsorbent polymer or the superabsorbent polymer material is contained that can be absorbed by respectively the superabsorbent polymer or the superabsorbent polymer material. The swelling capacity is measured at the swelling equilibrium. The swelling capacity is typically expressed in g/g (grams of swelling solution/grams of dry superabsorbent polymer or dry superabsorbent polymer material).

The swelling capacity is proportional to the swelling volume of the superabsorbent polymer gel at the swelling equilibrium.

The swelling volume of the superabsorbent polymer gel at the swelling equilibrium can be obtained via the *P-V* (Pressure-Swelling Volume) diagram of the tested superabsorbent polymer system (at constant temperature T) as explained hereinafter in detail.

The gel strength of a superabsorbent polymer may be characterized through the calculation of different output parameters such as the bulk modulus or the shear modulus of the superabsorbent polymer gel. The gel strength is typically determined at the swelling equilibrium volume, i.e. where the internal pressure equals the external pressure, as explained hereinafter in details.

• bulk modulus

The bulk modulus (*K*) of a superabsorbent polymer or a superabsorbent polymer material gel measures the resistance of respectively the superabsorbent polymer or the superabsorbent polymer material gel to uniform compression

The bulk modulus of a superabsorbent polymer gel can be obtained via the derivative of the *p-V*-diagram of the tested system (at constant temperature *T*):

$$K(T) = -V(\partial p/\partial V).$$

✔ Method for calculating the swelling capacity and/or the bulk modulus of a superabsorbent polymer gel at the swelling equilibrium

The swelling gel of a superabsorbent polymer has a swelling volume V and an internal pressure $P_{int}$. The liquid in which the swelling gel of a superabsorbent polymer is contained, i.e. the swelling liquid exerts an external pressure $P_{ext}$ onto the gel.

In this method, the swelling volume V of a superabsorbent polymer gel is varied by inputting increasing values $V_i$ for the swelling volume V. For each inputted swelling volume values $V_i$, the corresponding internal pressure values $P_{int, i}$ is calculated. A $P_{int, i} = f(V_i)$ diagram is then generated. The swelling capacity and the bulk modulus of the superabsorbent polymer are then calculated from the $P_{int, i} = f(V_i)$ diagram, the swelling capacity being proportional to the value of the swelling volume $V_{i\ (eq)}$ for which the internal pressure $P_{int}$ is equal to the external pressure $P_{ext}$ and the bulk modulus being equal to the slope of the $P_{int, i} = f(V_i)$ diagram determined at the swelling volume value $V_{i\ (eq)}$.

The swelling capacity ($C_0$) is calculated according to the following formula:

$$C_0 \text{ (Swelling capacity)} = (V_{i\ (eq)}\text{*}\ \rho_{water})/M$$

wherein $\rho_{water}$ is the water density and M is the mass of the dry superabsorbent polymer.

In some embodiments, wherein the superabsorbent polymer is a polyacrylate polymer which comprises 16 polyelectrolyte polymer chains of polymerized acrylate monomers connected to 8 tetra-functional cross-linker molecules, M is calculated according to the following formula:

$$M = \frac{16NM_{PA} + 8M_{CL}}{N_A} \frac{1}{(1 - \rho_W)}$$

wherein N is the length of the polymer chain (N = 25-300), $M_{PA}$ is the average molecular mass of the acrylate monomers taking into account the degree of neutralization ($M_{PA}$ = 94 / 83 / 79.3 g/mol for 100% / 50% / 33% neutralization), $M_{CL}$ is the molecular mass of the cross-linker molecules ($M_{CL}$ = 500 g/mol), and $\rho_W$ is the percentage of residual water which is typically equal to 0.5% and $N_A$ is the Avogadro Number.

In some embodiments, the first input value set and the second input value set inputted into the coarse-grained molecular dynamics model comprise the values of the cross-linker density. In such embodiments, the values of the swelling capacity and the bulk modulus can be calculated in response to each value of the cross-linker density which is inputted into the model. A K= f($C_0$) diagram can then be generated. This diagram represented the so-called capacity/gel strength trade off curve representing the variation of the gel strength (represented by the bulk modulus) with the absorption capacity. This diagram will help to understand the influence that the molecular parameter(s) of a superabsorbent polymer may have on its swelling capacity and bulk modulus. The person skilled in the art will be able to determine from the diagram the suitable molecular parameter values that a superabsorbent polymer material needs to have in order to exhibit the best performances.

• shear modulus

The shear modulus of a superabsorbent polymer or a superabsorbent polymer material gel measures the resistance of respectively the superabsorbent polymer or the superabsorbent polymer material gel to shearing strains.

V Method for calculating the shear modulus

In some embodiments, the shear modulus of a superabsorbent polymer gel is determined at the swelling equilibrium by inputting different values x; for the shear strain of the superabsorbent polymer and calculating the corresponding shear stress $y_i$ for each value $x_i$. The volume of the superabsorbent polymer gel of the model is maintained constant. A $y_i= f(x_i)$ diagram is then generated. The shear modulus is calculated from the generated diagram, the shear modulus being equal to the slope of the $y_i= f(x_i)$ diagram. Alternatively, the shear modulus G of the superabsorbent polymer gel can be calculated via the following formula, wherein v represents the Poisson's ratio of the material:

$$G = \frac{3(1 - 2\nu)}{2(1 + \nu)}K$$

wherein K is the bulk modulus of the superabsorbent polymer gel.

In some embodiments, the poisson's ratio of the superabsorbent polymer gel may be of 0.45. In such embodiments, the shear modulus G will be G ≈ 0.1 K.

In the method of the present invention, only the variation between different values of the same first and second performance output parameters is determined. For example, if one of the first performance output parameter is the swelling capacity, one of the second performance output parameter has to also be the swelling capacity.

Software

The coarse-grained molecular dynamics model is formulated and all the calculations are performed by using the software package ESPRESSO or LAMMPS.

The software package ESPRESSO used is the ESPRESSO version 2.1.2g, MPI for Polymer Research, Mainz, Germany, available from http://espressowiki.mpip-mainz.mpg.de.

The software package LAMMPS used is the LAMMPS version June 27, 2010, available from http://lammps.sandia.gov/.

A computer system is used for operating the coarse-grained molecular dynamics model. The computer system may comprise a central processing unit, a graphical user interface including a display communicatively coupled to the central processing unit, and a user interface selection device communicatively coupled to the central processing unit. The user interface selection device can be used to input data and information into the central processing unit. The central processing unit can include or has access to memory or data storage units, e.g., hard drive(s), compact disk(s), tape drive(s), and similar memory or data storage units for storing various data and inputs which can be accessed and used in operating the coarse-grained molecular dynamics model. Central processing unit can be part of a SUN workstation running a UNIX® operating system, part of a personal computer using INTEU® PC architecture and running a MICROSOFT WINDOWS® operating system, or part of another similarly capable computer architecture and accompanying operating system.

A superabsorbent polymer is displayed by the software in a cubic simulation cell 3 as for example shown in Figure 2. In some embodiments, as for example shown in Figure 2, the cubic simulation cell 3 comprises a superabsorbent polymer material 4 which is contained in a saline solution. In some of these embodiments, as for example shown on Figure 2, the superabsorbent polymer comprised in the cubic simulation cell 3 comprises 16 polyelectrolyte polymer chains 5 of polymerized monomers connected to 8 tetra-functional cross-linker molecules 6. The cubic simulation cell 3 further comprises the sodium counterions 7 and the chloride co-ions 8 of the saline solution.

In some embodiments, the method according to the present invention may further comprise additional validation steps in order to verify if the variation between the value(s) of the one or more first performance output parameter(s) and the value(s) of the one or more second performance parameter(s) which is determined by the method is in accordance with what is observed in reality.

In such embodiments, a first and a second superabsorbent polymer material may be obtained by any polymerization process known by the person skilled in the art. The value(s) of the same one or more first molecular parameter(s) as the one or more first molecular parameter(s) inputted into the coarse-grained molecular dynamics model are measured by analytical methods for the first superabsorbent polymer material and the value(s) of the same one or more second molecular parameter(s) as the one or more second molecular parameter(s) inputted into the coarse-grained molecular dynamics model are also measured by analytical methods for the second superabsorbent polymer material.

The value(s) of the one or more first performance output parameter(s) of the first superabsorbent polymer material and the value(s) of the one or more second performance output parameter(s) of the second superabsorbent polymer material are measured by analytical methods.

The variation between the value(s) of the one or more first performance output parameter(s) and the value(s) of the one or more second performance output parameter(s) measured by analytical methods is then determined and compare with the variation between the value(s) of the one or more first performance output parameter(s) and the value(s) of the one or more second performance output parameter(s) determined by the simulation.

The variation between the value(s) of the one or more first performance output parameter(s) and the value(s) of the one or more second performance output parameter(s) measured by analytical methods is determined between different values of the same first and second performance output parameter.

With one of the first and one of the second molecular parameter(s) being the cross-linker density and/or the percentage of dangling chains and/or the functionality of the cross-linker molecules , the value(s) of respectively the cross-linker density and/or the percentage of dangling chains and/or the functionality of the cross-linker molecules can be measured by using NMR including 1H-NMR or 13C-NMR including MAS (Magic Angle Spinning) technique as know by the person skilled in the art.

In some embodiments, wherein one of the first and one of the second molecular parameter(s) are the degree of neutralization, the value(s) of the degree of neutralization can be measured by acid/base titration as known by the person skilled in the art.

In some embodiments, wherein one of the first and one of the second molecular parameter(s) are the percentage of extractable, the percentage of extractable can be measured according to EDANA method WSP 270.2 (05).

In some embodiments wherein one of the first and one of the second performance output parameter(s) are the swelling capacity $C_0$, the value(s) of the swelling capacity $C_0$ can be measured according to Cylinder Centrifuge Retention Capacity (CCRC) method described on pages 70 and 71 of WO 2006/083585 A2.

In some embodiments wherein one of the first and one of the second performance output parameter(s) are the shear modulus, the value(s) of the shear modulus G of a swollen gel of a superabsorbent polymer material, especially of a polyacrylate superabsorbent polymer material can be calculated according to the following equation which corresponds to a modified version of the equation 5.34 disclosed on page 203 of "Modern Superabsorbent Polymer Technology", Frederic L. Buchholz, Andrew T. Graham, Wiley-VCH, Edition 1998:

$$G = \frac{P}{\left( \dfrac{C_0}{1.02 * Q_{eq}} \right)^{\frac{1}{0.44}} - 1}$$

with $Q_{eq}= 1.1 * Q_{(96h)}$

wherein $C_0$ is the value of the swelling capacity, $Q_{eq}$ is the value of the Absorption Against Pressure of the superabsorbent polymer material measured at the swelling equilibrium, $Q_{(96h)}$ is the value of the Absorption Against Pressure after 96h (96h-AAP) of the superabsorbent polymer material as measured according to the 96h-AAP test method herein disclosed and P is the value of the pressure which is applied to the sample of the superabsorbent polymer material which is tested according to the 96h-AAP test method. According to the 96h-AAP test method herein disclosed, the value of the applied pressure is P= 0.7 psi (4.83 kPa).

In some embodiments, additional superabsorbent polymer materials having different molecular parameter values may be used for the validation steps.

The method according to the present invention is particularly advantageous since it is possible to predict the influence that the molecular parameter(s) of superabsorbent polymer materials may have on the performances parameter(s) of such superabsorbent polymer material(s) in a time- and cost-effective manner since no synthesis of superabsorbent polymer materials having different values for the molecular parameters needs to be made.

Furthermore, the influence that the molecular parameter(s) of superabsorbent polymer materials may have on the performances parameter(s) of such superabsorbent polymer material(s) can be determined in a more accurate manner since a high number of different values for each molecular parameter can be inputted into the model. It would not be possible to synthesize the corresponding amount of superabsorbent polymer materials with different values of molecular parameter(s) since this would considerably increase the development costs.

Once the targeted output parameter value(s) has/have been determined, the superabsorbent polymer material with the corresponding molecular parameter value(s) can then be synthesized. Therefore, the method according to the present invention can be used for obtaining superabsorbent polymer material suitable for use in absorbent articles in a time- and cost-effective manner.

TEST METHOD

96h-AAP Test Method

This method determines the capacity of superabsorbent polymer materials to absorb saline solution under a specified pressure applied for 96h.

The procedure of the EDANA Absorption Under Pressure Test Method 442.2-02 has been followed with the following modifications:

- Section 4 has been replaced by the following:
  4. Principle
  The test portion is weighed and spread on the bottom filter screen closing a specified cylinder. A uniform pressure is applied on the test portion. The cylinder is then placed on a filter plate, which is placed in a Petri dish filled with saline solution. After an absorption contact time of 96 hours, the cylinder is removed from the filter plate and weighed to determine the amount of fluid absorbed.

- The following section 6.5 has been added:
  6.5 12 L Square plastic tub as available from VWR International GmbH, Darmstadt, Germany under the article reference CORAG606221.

- Section 6.1.5 is replaced by the following:
  6.1.5 Plastic piston, with a cylindrical weight, of which the total mass is equal to (1394 $\pm$ 5) g. The piston diameter, d2, is such that d1-d2 = (0.8 $\pm$ 0.2) mm, and the height of the cylindrical weight is (60,0 $\pm$ 0.5) mm.

- Section 8.1 is replaced by the following:
  8.1 Weigh, to the nearest 0.0005 g, a 0.400 g test portion of PA superabsorbent powder test sample and record the mass, $m_S$.

- Section 8.7 is replaced by the following:

  8.7.1 Place the cylinder apparatus (piston and cylinder) on the damp filter paper simultaneously adding the weight to the apparatus and cover the Petri dish and the complete apparatus (cylinder apparatus and weight) with the plastic tub to avoid the evaporation of the sodium chloride solution. Allow the test portion to absorb the saline solution for 24h $\pm$ 0.5 h
  8.7.2 Remove the plastic tub and lift the complete apparatus (cylinder apparatus and weight)
  8.7.3 Discard the sodium chloride solution contained in the Petri dish and the filter paper 8.7.4 Repeat steps 8.5 and 8.6 wherein a new round filter paper is placed on the filter plate.
  8.7.5 Replace the complete apparatus (cylinder apparatus and weight) on the new damp filter paper and cover the Petri dish and the complete apparatus (cylinder apparatus and weight) with the plastic tub to avoid the evaporation of the sodium chloride solution. Allow the test portion to absorb the saline solution for another 24h $\pm$ 0.5 h.
  8.7.6 Repeat steps 8.7.2 to 8.7.5 twice until the absorption contact time of 96h is reached.

- Section 9 is replaced by the following:
  For each portion, calculate the absorption against pressure after 96h (96h-AAP) expressed as a mass fraction in g/g:

  $$Q_{(96h)} = \frac{m_B - m_A}{m_S}$$

  Where:

  $m_S$ is the mass, expressed in grams, of the dry test portion
  $m_A$ is the mass, expressed in grams, of dry cylinder apparatus
  $m_B$ is the mass, expressed in grams, of the cylinder apparatus after suction

Take the average of the 2 calculated values.

**Claims**

1. A method for determining the performances of a superabsorbent polymer comprising the steps of:

   a) inputting the value(s) of one or more first molecular parameter(s) of the superabsorbent polymer into a coarse-grained molecular dynamics model; and calculating the value(s) of one or more first performance output parameter(s); and
   b) inputting the value(s) of one or more second molecular parameter(s) of the superabsorbent polymer into said coarse-grained molecular dynamics model; and calculating the value(s) of one or more second performance output parameter(s); and
   c) determining the variation between the value(s) of the one or more first performance output parameter(s) and the value(s) of the one or more second performance output parameter(s);

   wherein a computer system is used for operating the coarse-grained molecular dynamics model; and
   wherein the method is used for obtaining superabsorbent polymer materials suitable for use in absorbent articles; and further
   wherein the value(s) of one or more first molecular parameter(s) inputted in step a), form a first input value set of a first molecular parameter set, and the value(s) of one or more second molecular parameter(s) inputted in step b), form a second input value set of a second molecular parameter set; and
   wherein said first and second molecular parameter sets are the same and comprise molecular parameters selected from the group consisting of: cross-linker density, polydispersity index, percentage of dangling chains, degree of neutralization, functionality of the cross-linker molecules, percentage of extractable, molecular weight of the monomers and combinations thereof, and

wherein the value(s) of one or more first performance output parameters calculated in step a), form a first output value set of a first output parameter set, and the value(s) of one or more second performance output parameters calculated in step b), form a second output value set of a second output parameter set, and wherein said first and second output parameter sets comprise performance output parameters selected from the group consisting of: i) swelling capacity, ii) bulk modulus, iii) shear modulus and combinations thereof,
wherein the method further comprises the steps of:

a2) obtaining a first superabsorbent polymer material; and measuring analytically the value(s) of said one or more first molecular parameter(s) of said first superabsorbent polymer material; measuring analytically the value(s) of said one or more first performance output parameter(s); and
b2) obtaining a second superabsorbent polymer material; and measuring analytically the value(s) of said one or more second molecular parameter(s) of said second superabsorbent polymer material; measuring analytically the value(s) of said one or more second performance output parameter(s); and
c2) determining the variation between the value(s) of the one or more first performance output parameter(s) measured in step a2) and the value(s) of the one or more second performance output parameter(s) measured in step b2); and

d) comparing the variation determined in step c2) with the variation determined in step c).

2. The method according to claim 1, wherein the performances of the superabsorbent polymer are determined for a swollen gel of the superabsorbent polymer which is contained in a liquid selected from deionized water or a saline solution.

3. The method according to claim 1 or 2, wherein said first and second input value sets are within the ranges of: a cross-linker density of 0.01 to 2 mol%; a polydispersity index of 1 to 5; a percentage of dangling chains of 0 to 50%; a degree of neutralization of 0 to 100 mol%, preferably 50 to 100 mol%; tetra-functional, hexa-functional or octafunctional cross-linker molecules, a percentage of extractable of 0 to 50%, a molecular weight of the monomers of 28 to 72 g/mol.

4. The method according to claim 3, wherein the first input value set and the second input value set inputted in step a) and b) respectively comprise the values of the cross-linker density.

5. The method according to claim 4, wherein the swelling gel of the superabsorbent polymer has a swelling volume $V$ and an internal pressure $P_{int}$; and

wherein the liquid in which the swelling gel of the superabsorbent polymer is contained exerts an external pressure $P_{ext}$ onto the superabsorbent polymer gel; and
wherein the values of the swelling capacity and the bulk modulus of the superabsorbent polymer are calculated by:

i) inputting increasing values $V_i$ for the swelling volume $V$; and
ii) calculating the corresponding internal pressure values $P_{int}$ for each value $V_i$ inputted in step i)
iii) generating a $P_{int, i} = f(V_i)$ diagram; and
iv) calculating the values of the swelling capacity and the bulk modulus from the diagram generated in step iii), the value of the swelling capacity of the superabsorbent polymer being proportional to the value of the swelling volume $V_{i\,(eq)}$ for which the internal pressure $P_{int}$ is equal to the external pressure $P_{ext}$ and the value of the bulk modulus being equal to the slope of the $P_{int, i} = f(V_i)$ diagram generated in step iii) at $V_i = V_{i(eq)}$.

6. The method according to claim 5, wherein the value of the shear modulus of the superabsorbent polymer is calculated by:

i) inputting increasing values $x_i$ for the shear strain of the superabsorbent polymer having a swelling volume value $V_i = V_{i(eq)}$; and
ii) calculating the corresponding shear stress $y$; for each value $x_i$ inputted in step i)
iii) generating a $y_i = f(x_i)$ diagram
iv) calculating the value of the shear modulus from the diagram generated in step iii), the shear modulus being equal to the slope of the $y_i = f(x_i)$ diagram generated in step iii).

7. The method according to any of the preceding claims, wherein the superabsorbent polymer comprises 16 polyelectrolyte polymer chains of polymerized monomers connected to 8 tetra-functional cross-linker molecules and counterions,

wherein the polymerized monomers of the polyelectrolyte chains interact with one another intra and inter molecular, with the cross-linker molecules and with the counterions via a truncated and shifted Lennard-Jones or Weeks-Chandler-Anderson potential as defined in the following formula:

$$U_{\mathrm{LJ}}(r_{ij} < r_{\mathrm{cut}}) = 4\epsilon \left[ \left(\frac{\sigma}{r_{ij}}\right)^{12} - \left(\frac{\sigma}{r_{ij}}\right)^{6} + \frac{1}{4} \right] \quad \text{(F1)}$$

and wherein the polymerized monomers of a same polyelectrolyte polymer chain interact with one another and with the cross-linker molecules to which the chain is connected via a FENE (Finitely Extensible Nonlinear Elastic) potential as defined in the following formula

$$U(r_{ij}) = -\frac{1}{2}KR_{\mathrm{max}}^{2}\ln\left[1 - \left(\frac{r_{ij} - r_0}{R_{\mathrm{max}}}\right)^{2}\right] \quad \text{(F2)}$$

and wherein the polymerized monomers of the polyelectrolyte polymer chains of the model interact with one another intra and inter molecular and with the counterions and wherein the counterions interact with one another via a coulomb potential as defined in the following formula:

$$U_{\mathrm{C}}(r_{ij}) = l_B k_B T \frac{q^2}{r_{ij}} \quad \text{(F3)}$$

8. The method according to any of the preceding claims, wherein the superabsorbent polymer is a partially neutralized polyacrylic acid and/or polyacrylate polymer.

**Patentansprüche**

1. Verfahren zum Bestimmen der Leistungen eines Superabsorber-Polymers, umfassend die Schritte:

a) Eingeben des/der Werte(s) eines oder mehrerer erster molekularer Parameter des Superabsorber-Polymers in ein grobkörnig molekulardynamisches Modell; und Berechnen des/der Werte(s) eines oder mehrerer erster Leistungsausgabeparameter; und
b) Eingeben des/der Werte(s) eines oder mehrerer zweiter molekularer Parameter des Superabsorber-Polymers in das grobkörnig molekulardynamische Modell; und Berechnen des/der Werte(s) eines oder mehrerer zweiter Leistungsausgabeparameter; und
c) Bestimmen der Abweichung zwischen dem/den Wert(en) des einen oder der mehreren ersten Leistungsausgabeparameter und dem/den Wert(en) des einen oder der mehreren zweiten Leistungsausgabeparameter;

wobei zum Betreiben des grobkörnig molekulardynamischen Modells ein Computersystem verwendet wird; und
wobei das Verfahren zum Erhalten von Superabsorber-Polymermaterialien verwendet wird, die zum Gebrauch in Absorptionsartikeln geeignet sind; und ferner,
wobei der/die Wert(e) eines oder mehrerer erster molekularer Parameter, die in Schritt a) eingegeben werden, einen ersten Eingabewertsatz eines ersten molekularen Parametersatzes ausbilden, und der/die Wert(e) eines oder mehrerer zweiter molekularer Parameter, die in Schritt b) eingegeben werden, einen zweiten Eingabewertsatz eines zweiten molekularen Parametersatzes ausbilden; und

wobei der erste und der zweite molekulare Parametersatz derselbe sind und molekulare Parameter umfassen, die aus der Gruppe ausgewählt sind, bestehend aus: Vernetzerdichte, Polydispersitätsindex, Prozentsatz hängender Ketten, Neutralisationsgrad, Funktionalität der Vernetzermoleküle, Prozentsatz an Extrahierbarem, Molekulargewicht der Monomere und Kombinationen davon, und

wobei der/die Wert(e) eines oder mehrerer in Schritt a) berechneter erster Leistungsausgabeparameter einen ersten Ausgabewertsatz eines ersten Ausgabeparametersatzes ausbilden und der/die Wert(e) eines oder mehrerer in Schritt b) berechneter zweiter Leistungsausgabeparameter einen zweiten Ausgabewertsatz eines zweiten Ausgabeparametersatzes ausbilden, und wobei der erste und der zweite Ausgabeparametersatz Leistungsausgabeparameter umfassen, die aus der Gruppe ausgewählt sind, bestehend aus: i) Quellvermögen, ii) Kompressionsmodul, iii) Schermodul und Kombinationen davon,

wobei das Verfahren ferner die Schritte umfasst:

a2) Erhalten eines ersten Superabsorber-Polymermaterials; und analytisches Messen des/der Werte(s) des einen oder der mehreren ersten molekularen Parameter(s) des ersten Superabsorber-Polymermaterials; analytisches Messen des/der Werte(s) des einen oder der mehreren ersten Leistungsausgabeparameter(s); und

b2) Erhalten eines zweiten Superabsorber-Polymermaterials; und analytisches Messen des/der Werte(s) des einen oder der mehreren zweiten molekularen Parameter(s) des zweiten Superabsorber-Polymermaterials; analytisches Messen des/der Werte(s) des einen oder der mehreren zweiten Leistungsausgabeparameter(s); und

c2) Bestimmen der Abweichung zwischen dem/den Wert(en) des einen oder der mehreren ersten Leistungsausgabeparameter(s), die in Schritt a2) gemessen werden, und dem/den Wert(en) des einen oder der mehreren zweiten Leistungsausgabeparameter(s), die in Schritt b2) gemessen werden; und

d) Vergleichen der in Schritt c2) bestimmten Abweichung mit der in Schritt c) bestimmten Abweichung.

2. Verfahren nach Anspruch 1, wobei die Leistungen des Superabsorber-Polymers für ein gequollenes Gel des Superabsorber-Polymers bestimmt werden, das in einer Flüssigkeit enthalten ist, die aus entionisiertem Wasser oder einer Salzlösung ausgewählt ist.

3. Verfahren nach Anspruch 1 oder 2, wobei der erste und der zweite Eingabewertsatz innerhalb der Bereiche liegen von: einer Vernetzerdichte von 0,01 bis 2 Mol-%; einem Polydispersitätsindex von 1 bis 5; einem Prozentsatz hängender Ketten von 0 bis 50 %; einem Neutralisationsgrad von 0 bis 100 Mol-%, vorzugsweise 50 bis 100 Mol-%; tetrafunktionellen, hexafunktionellen oder octafunktionellen Vernetzermolekülen, einem Prozentsatz von Extrahierbarem von 0 bis 50 %, einem Molekulargewicht der Monomere von 28 bis 72 g/mol.

4. Verfahren nach Anspruch 3, wobei der erste Eingabewertsatz und der zweite Eingabewertsatz, die in Schritt a) und b) eingegeben werden, jeweils die Werte der Vernetzerdichte umfassen.

5. Verfahren nach Anspruch 4, wobei das Quellgel des Superabsorber-Polymers ein Quellvolumen $V$ und einen Innendruck $P_{int}$ aufweist; und

wobei die Flüssigkeit, in der das Quellgel des Superabsorber-Polymers enthalten ist, einen Außendruck $P_{Auß}$, auf das Superabsorber-Polymergel ausübt; und

wobei die Werte der Quellkapazität und des Kompressionsmoduls des Superabsorber-Polymers berechnet werden durch:

i) Eingeben von steigenden Werten $V_i$ für das Quellvolumen $V$; und

ii) Berechnen der entsprechenden Innendruckwerte $P_{Inn}$ für jeden in Schritt i) eingegebenen Wert $V_i$

iii) Erzeugen eines Diagramms für $P_{Inn, i} = f(V_i)$; und

iv) Berechnen der Werte der Quellkapazität und des Kompressionsmoduls aus dem in Schritt iii) erzeugten Diagramm, wobei der Wert der Quellkapazität des Superabsorber-Polymers proportional zu dem Wert des Quellvolumens $V_{i(eq)}$ ist, für den der Innendruck $P_{Inn}$ gleich dem Außendruck $P_{Auß}$ ist, und der Wert des Kompressionsmoduls gleich der Steigung des in Schritt iii) bei $V_i = V_{i(eq)}$ erzeugten Diagramms $P_{Inn, i} = f(V_i)$ ist.

6. Verfahren nach Anspruch 5, wobei der Wert des Schermoduls des Superabsorber-Polymers berechnet wird durch:

i) Eingeben von steigenden Werten $x_i$ für die Scherdehnung des Superabsorber-Polymers, das einen Quellvolumenwert $V_i = V_{i(eq)}$ aufweist; und

ii) Berechnen der entsprechenden Scherkraft $y_i$ für jeden in Schritt i) eingegebenen Wert $x_i$

iii) Erzeugen eines Diagramms für $y_i = f(x_i)$

iv) Berechnen des Wertes des Schermoduls aus dem in Schritt iii) erzeugten Diagramm, wobei der Schermodul gleich der Steigung des in Schritt iii) erzeugten Diagramms $y_i = f(x_i)$ ist.

7. Verfahren nach einem der vorstehenden Ansprüche, wobei das Superabsorber-Polymer 16 Polyelektrolytpolymerketten aus polymerisierten Monomeren umfasst, die mit 8 tetrafunktionellen Vernetzermolekülen und Gegenionen verbunden sind,

wobei die polymerisierten Monomere der Polyelektrolytketten intra- und intermolekular miteinander, mit den Vernetzermolekülen und mit den Gegenionen über ein abgeschnittenes und verschobenes Lennard-Jones- oder Weeks-Chandler-Anderson-Potential interagieren, wie in der folgenden Formel definiert:

$$U_{LJ}(r_{ij} < r_{Grenz}) = 4\epsilon \left[ \left( \frac{\sigma}{r_{ij}} \right)^{12} - \left( \frac{\sigma}{r_{ij}} \right)^{6} + \frac{1}{4} \right] \quad (F1)$$

und wobei die polymerisierten Monomere einer gleichen Polyelektrolytpolymerkette miteinander und mit den Vernetzermolekülen, an die die Kette gebunden ist, über ein FENE-Potential (begrenzt dehnbares, nichtlineares elastisches Potential) interagieren, wie in der folgenden Formel definiert

$$U(r_{ij}) = -\frac{1}{2} K R_{Max.}^2 \ln \left[ 1 - \left( \frac{r_{ij} - r_0}{R_{Max.}} \right)^2 \right] \quad (F2)$$

und wobei die polymerisierten Monomere der Polyelektrolytpolymerketten des Modells intra- und intermolekular miteinander und mit den Gegenionen interagieren und wobei die Gegenionen über ein Coulomb-Potential miteinander interagieren, wie in der folgenden Formel definiert:

$$U_C(r_{ij}) = l_B k_B T \frac{q^2}{r_{ij}} \quad (F3)$$

8. Verfahren nach einem der vorstehenden Ansprüche, wobei das Superabsorber-Polymer ein teilweise neutralisiertes Polyacrylsäure- und/oder Polyacrylatpolymer ist.

## Revendications

1. Procédé permettant de déterminer les performances d'un polymère superabsorbant comprenant les étapes consistant à :

a) introduire la ou les valeurs d'un ou de plusieurs premiers paramètres moléculaires du polymère superabsorbant dans un modèle de dynamique moléculaire à grain grossier ; et calculer la ou les valeurs d'un ou de plusieurs premiers paramètres de sortie de performance ; et

b) introduire la ou les valeurs d'un ou de plusieurs seconds paramètres moléculaires du polymère superabsorbant dans ledit modèle de dynamique moléculaire à grain grossier ; et calculer la ou les valeurs d'un ou de plusieurs seconds paramètres de sortie de performance ; et

c) déterminer la variation entre la ou les valeurs du ou des premiers paramètres de sortie de performance et la ou les valeurs du ou des seconds paramètres de sortie de performance ;

dans lequel un système informatique est utilisé pour faire fonctionner le modèle de dynamique moléculaire à grain grossier ; et

dans lequel le procédé est utilisé pour obtenir des matériaux polymères superabsorbants destinés à être utilisés dans des articles absorbants ; et en outre

dans lequel la ou les valeurs d'un ou de plusieurs premiers paramètres moléculaires introduites à l'étape a), forment un premier ensemble de valeurs d'entrée d'un premier ensemble de paramètres moléculaires, et la ou les valeurs d'un ou de plusieurs seconds paramètres moléculaires introduites à l'étape b), forment un second ensemble de valeurs d'entrée d'un second ensemble de paramètres moléculaires ; et

dans lequel lesdits premier et second ensembles de paramètres moléculaires sont identiques et comprennent des paramètres moléculaires choisis dans le groupe constitué par : une densité de réticulation, un indice de polydispersité, un pourcentage de chaînes pendantes, un degré de neutralisation, une fonctionnalité des molécules de réticulation, un pourcentage d'un produit extractible, un poids moléculaire des monomères et des combinaisons de ceux-ci, et

dans lequel la ou les valeurs d'un ou de plusieurs premiers paramètres de sortie calculés à l'étape a) forment un premier ensemble de valeurs de sortie d'un premier ensemble de paramètres de sortie, et la ou les valeurs d'un ou de plusieurs seconds paramètres de sortie calculés à l'étape b) forment un second ensemble de valeurs de sortie d'un second ensemble de paramètres de sortie, et dans lequel lesdits premier et second ensembles de paramètres de sortie comprennent des paramètres de sortie de performance choisis dans le groupe constitué par : i) une capacité de gonflement, ii) un module de compression, iii) un module de cisaillement et des combinaisons de ceux-ci,

dans lequel le procédé comprend en outre les étapes consistant à :

> a2) obtenir un premier matériau polymère superabsorbant ; et mesurer analytiquement la ou les valeurs dudit ou desdits premiers paramètres moléculaires dudit premier matériau polymère superabsorbant ; mesurer analytiquement la ou les valeurs dudit ou desdits premiers paramètres de sortie de performance ; et
>
> b2) obtenir un second matériau polymère superabsorbant ; et mesurer analytiquement la ou les valeurs dudit ou desdits seconds paramètres moléculaires dudit second matériau polymère superabsorbant ; mesurer analytiquement la ou les valeurs dudit ou desdits seconds paramètres de sortie de performance ; et
>
> c2) déterminer la variation entre la ou les valeurs du ou des premiers paramètres de sortie de performance mesurés à l'étape a2) et la ou les valeurs du ou des seconds paramètres de sortie de performance mesurés à l'étape b2) ; et

d) comparer la variation déterminée à l'étape c2) à la variation déterminée à l'étape c).

2. Procédé selon la revendication 1, dans lequel les performances du polymère superabsorbant sont déterminées pour un gel gonflé du polymère superabsorbant qui est contenu dans un liquide choisi parmi l'eau désionisée ou une solution saline.

3. Procédé selon la revendication 1 ou 2, dans lequel lesdits premier et second ensembles de valeurs d'entrée se situent dans les plages de : une densité de réticulation de 0,01 à 2 % en moles ; un indice de polydispersité de 1 à 5 ; un pourcentage de chaînes pendantes compris entre 0 et 50 % ; un degré de neutralisation de 0 à 100 % en moles, de préférence de 50 à 100 % en moles ; molécules de réticulation tétra-fonctionnelles, hexa-fonctionnelles ou octafonctionnelles, un pourcentage d'un produit extractible de 0 à 50 %, un poids moléculaire des monomères de 28 à 72 g/mol.

4. Procédé selon la revendication 3, dans lequel le premier ensemble de valeurs d'entrée et le second ensemble de valeurs d'entrée introduites aux étapes a) et b) respectivement comprennent les valeurs de la densité de réticulation.

5. Procédé selon la revendication 4, dans lequel le gel de gonflement du polymère superabsorbant a un volume de gonflement V et une pression interne $P_{int}$ ; et

le liquide dans lequel le gel de gonflement du polymère superabsorbant est contenu exerce une pression externe $P_{ext}$ sur le gel de polymère superabsorbant ; et
dans lequel les valeurs de la capacité de gonflement et du module de compression du polymère superabsorbant sont calculées par :

i) l'introduction de valeurs croissantes V i pour le volume de gonflement V ; et

ii) le calcul des valeurs de pression interne $P_{int}$ correspondantes pour chaque valeur $V_i$ introduite à l'étape i)

iii) la génération d'un diagramme $P_{int, i} = f(V_i)$ ; et

iv) le calcul des valeurs de la capacité de gonflement et du module de compression à partir du diagramme généré à l'étape iii), la valeur de la capacité de gonflement du polymère superabsorbant étant proportionnelle à la valeur du volume de gonflement $V_{i (eq)}$ pour lequel la pression interne $P_{int}$ est égale à la pression externe $P_{ext}$ et la valeur du module de compression étant égale à la pente du diagramme $P_{int, i} = f(V_i)$ généré à l'étape iii) à $V_i = V_{i(eq)}$.

6. Procédé selon la revendication 5, dans lequel la valeur du module de cisaillement du polymère superabsorbant est calculée par :

i) l'introduction des valeurs croissantes $x_i$ pour la contrainte de cisaillement du polymère superabsorbant ayant une valeur de volume de gonflement $V_i = V_{i(eq)}$ ; et

ii) le calcul de la contrainte de cisaillement correspondante $y_i$ pour chaque valeur $x_i$ introduite à l'étape i)

iii) la génération d'un diagramme $y_i = f(x_i)$

iv) le calcul de la valeur du module de cisaillement à partir du diagramme généré à l'étape iii), le module de cisaillement étant égal à la pente du diagramme $y_i = f(x_i)$ généré à l'étape iii).

7. Procédé selon l'une quelconque des revendications précédentes, dans lequel le polymère superabsorbant comprend 16 chaînes polymères polyélectrolytes de monomères polymérisés reliés à 8 molécules de réticulation tétra-fonctionnelles et à des contre-ions,

dans lequel les monomères polymérisés des chaînes de polyélectrolytes interagissent entre eux au niveau intra et inter moléculaire, avec les molécules de réticulation et avec les contre-ions par l'intermédiaire d'un potentiel de Lennard-Jones ou de Weeks-Chandler-Anderson tronqué et décalé, tel que défini dans la formule suivante :

$$U_{LJ}\left(r_{ij} < r_{\text{couper}}\right) = 4 \in \left[ \left(\frac{\sigma}{r_{ij}}\right)^{12} - \left(\frac{\sigma}{r_{ij}}\right)^{6} + \frac{1}{4} \right] \qquad (F1)$$

et dans lequel les monomères polymérisés d'une même chaîne de polymère polyélectrolyte interagissent entre eux et avec les molécules de réticulation auxquelles la chaîne est reliée par l'intermédiaire d'un potentiel FENE (Finitely Extensible Nonlinear Elastic) tel que défini dans la formule suivante

$$U\left(r_{ij}\right) = -\frac{1}{2} K R_{max^2} In \left[ 1 - \left(\frac{r_{ij} - r_0}{R_{max}}\right)^2 \right] \qquad (F2)$$

et dans lequel les monomères polymérisés des chaînes polymères polyélectrolytes du modèle interagissent entre eux au niveau intra et inter moléculaire et avec les contre-ions et dans lequel les contre-ions interagissent entre eux par l'intermédiaire d'un potentiel de coulomb tel que défini dans la formule suivante :

$$U_C\left(r_{ij}\right) = l_B k_B T \frac{q^2}{r_{ij}} \qquad (F3)$$

8. Procédé selon l'une quelconque des revendications précédentes, dans lequel le polymère superabsorbant est un polymère d'acide polyacrylique et/ou de polyacrylate partiellement neutralisé.

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006083585 A2

**Non-patent literature cited in the description**

- **ARNOLD A. et al.** Simulating Charged Systems with ESPRESSO. *Computer Simulations in Condensed Matter Systems: from Materials to Chemical Biology; Lecture Notes in Physics,* 2006, vol. 703, 193-222
- **MANN B. A. et al.** The swelling behavior of charged hydrogels. *Macromol. Symp., Special issue: Molecular Mobility and Order in Polymer Systems,* 2006, vol. 237 (1), 90-107

- **PADDING J. T. et al.** Time and length scales of polymer melts studies by coarse-grained molecular dynamics simulations. *J. Chem. Phys.,* 2002, vol. 117 (2), 925-943
- **MANN B. A. et al.** Swelling of polyelectrolyte networks. *The Journal of Chemical Physics,* 2005, vol. 122 (15), 154903, , 114
- **FREDERIC L. BUCHHOLZ ; ANDREW T. GRAHAM.** Modern Superabsorbent Polymer Technology. Wiley-VCH, 1998, 203